# EUROPEAN PATENT SPECIFICATION

(11) **EP 4 288 779 B1**
(45) Date of publication and mention of the grant of the patent: **20.11.2024**
(21) Application number: 22704952.5
(22) Date of filing: 27.01.2022
(51) Int. Cl.: G01N 33/564

(54) **PROCESS AND KIT FOR THE IN VITRO DIAGNOSIS OF THE ANTIPHOSPHOLIPID ANTIBODY SYNDROME**
VERFAHREN UND KIT ZUR IN-VITRO-DIAGNOSE DES ANTIPHOSPHOLIPID-ANTIKÖRPERSYNDROMS
PROCÉDÉ ET TROUSSE POUR LE DIAGNOSTIC IN VITRO DU SYNDROME DES ANTIPHOSPHOLIPIDES

(30) Priority: 03.02.2021 IT 202100002306
(43) Date of publication of application: 13.12.2023
(73) Proprietor: Università degli Studi di Roma "La Sapienza", 00185 Roma (IT)
(72) Inventor: SORICE, Maurizio, 00185 Roma (RM) (IT); MISASI, Roberta, 00185 Roma (RM) (IT); GAROFALO, Tina, 00185 Roma (RM) (IT); LONGO, Agostina, 00185 Roma (RM) (IT); CAPOZZI, Antonella, 00185 Roma (RM) (IT); MANGANELLI, Valeria, 00185 Roma (RM) (IT); RECALCHI, Serena, 00185 Roma (RM) (IT); RIITANO, Gloria, 00185 Roma (RM) (IT)
(74) Representative: Trupiano, Federica
(86) International application number: PCT/IB2022/050708
(87) International publication number: WO 2022/167901

(56) References cited:
- EP-A1- 0 600 088
- WO-A1-2020/097528
- WO-A2-2006/109312
- MISASI ROBERTA ET AL: "Molecular Mechanisms of "Antiphospholipid Antibodies" and Their Paradoxical Role in the Pathogenesis of "Seronegative APS"", INTERNATIONAL JOURNAL OF MOLECULAR SCIENCES, vol. 21, no. 21, 9 November 2020 (2020-11-09), Basel, CH, pages 8411, XP055844831, ISSN: 1661-6596, DOI: 10.3390/ijms21218411
- ARVIEUX J ET AL: "Platelet activating properties of murine monoclonal antibodies to beta 2-glycoprotein I", THROMBOSIS AND HAEMOSTASIS, SCHATTAUER GMBH, DE, vol. 70, no. 2, 2 August 1993 (1993-08-02), pages 336 - 341, XP009151728, ISSN: 0340-6245

## Description

### ABSTRACT

The present invention relates to a process to perform an *in vitro* diagnosis of the antiphospholipid antibody syndrome, carried out on isolated samples of the subject to be diagnosed. Furthermore, the present invention relates to a kit which allows to perform a non-invasive diagnosis of the antiphospholipid antibody syndrome from an isolated sample of the subject for whom such a diagnosis is made.

### STATE OF THE ART

The antiphospholipid antibody syndrome (APS) is an autoimmune disease whose main clinical manifestations are (arterial and/or venous) thrombosis and/or obstetric complications. This syndrome can exist alone (primary APS) or in association with other autoimmune diseases, among which the systemic lupus erythematosus (secondary APS).

In the APS there are auto-antibodies produced by the immune system of the person affected by such syndrome, which recognize *self* structures of the body. In other words, said auto-antibodies do not recognize antigens coming from outside or considered as harmful, but recognize and attack physiological structures such as phospholipids, proteins or protein/phospholipid complexes.

The diagnosis of APS is based on laboratory examinations (presence of anti-cardiolipin, Lupus Anticoagulant, anti-β2-glycoprotein I antibodies) and clinical manifestations (pregnancy diseases, venous or arterial thrombosis). The Lupus Anticoagulant (LA), anti-cardiolipin antibodies (aCL), and anti-p2-glycoprotein antibodies (aβ2-GPI) represent the currently available tools for the laboratory-diagnosis of the anti phospholipid antibody syndrome.

However, several researchers have described case histories of patients who have a clinical profile suggesting the antiphospholipid antibody syndrome but who test negative on all currently available diagnostic examinations. In other words, it has been observed that some people, although having the clinical manifestations of APS, are negative on the diagnostic examinations currently available and described above. For these cases, the term seronegative APS (SN-APS) has been proposed and is now in use.

One of the auto-antigens recognized by the auto-antibodies produced by the immune system of the person affected with antiphospholipid antibody syndrome is the β2-Glycoprotein I (β2-GPI), a phospholipid-binding protein. The anti-β2-GPI antibodies are the main auto-antibodies used for the diagnosis of APS. It has been observed that several post-translational modifications are responsible for the formation of cryptic epitopes in the β2-GPI protein. The post-translational modifications are modifications that involve a protein and consist of covalent modifications of a specific amino acid. They can also induce changes in the net charge of the protein, leading to conformational modifications in the tertiary and quaternary structure. A post-translational modification that the β2-GPI protein undergoes is the glycation. However, it has been observed that even the diagnostic examination based on the detection of glycated anti-β2GPI antibodies does not represent to date a test capable of detecting the disease in subjects who manifest the symptoms but are negative to all the available diagnostic examinations. An accurate diagnosis of the antiphospholipid antibody syndrome is particularly important, on the one hand, to avoid the onset of diffuse thrombotic episodes with multi-organ involvement and, on the other hand, to be able to start a treatment to prevent the thrombotic events.

Thus, it is necessary to identify a new biomarker specific for the antiphospholipid antibody syndrome (APS) that allows to diagnose said syndrome even in subjects with obvious clinical manifestations but who are negative to all of the diagnostic investigations currently available.

Furthermore, there is a need to develop a non-invasive process that allows a rapid diagnosis of antiphospholipid antibody syndrome (APS) even in the subjects who, although presenting the typical clinical manifestations of said syndrome, are negative to all of the currently available diagnosis methods for APS.

### OBJECTS OF THE INVENTION

Object of the present invention is to provide a specific biomarker for the diagnosis of the anti phospholipid antibody syndrome (APS).

Another object of the present invention is to provide a rapid and non-invasive process which allows to diagnose the antiphospholipid antibody syndrome (APS).

A further object of the present invention is to provide a process which allows to diagnose the antiphospholipid antibody syndrome (APS) in subjects having the clinical manifestations of said syndrome but who test negative on all of the currently available diagnostic examinations.

Finally, object of the present invention is also to provide a kit which allows to carry out a non-invasive process to diagnose the antiphospholipid antibody syndrome (APS).

### DESCRIPTION OF THE INVENTION

The above purposes, as well as other purposes, are achieved by means of the object of the present invention, namely a process to perform an *in vitro* diagnosis of antiphospholipid antibody syndrome (APS), comprising the steps of:
a) having available one or more isolated biological samples that have been obtained from the subject to be diagnosed; and
b) determining, within said one or more isolated samples according to step a), the presence of antibodies directed towards the carbamylated P2-glycoprotein I.

In the present invention, the antiphospholipid antibody syndrome may be designated by the "APS" acronym. The antiphospholipid antibody syndrome is an acquired thrombophilia, characterized by thromboembolic events (causing stroke or transient ischemic attacks, myocardial infarction, limb ischemia) and/or pregnancy diseases. The antiphospholipid antibody syndrome is an autoimmune disease, in which the immune system of the subject affected by said disease produces antibodies that recognize and attack components of the human body. Said antibodies, which recognize *self* molecules, are referred by the term "auto-antibodies".

The process of the invention comprises detecting the presence, in the isolated biological samples of the subject for whom the diagnosis is to be made, of antibodies directed against the carbamylated form of the β2-glycoprotein I, said antibodies being auto-antibodies as defined above.

The carbamylation of the β2-glycoprotein I is a post-translational modification that P2-glycoprotein I protein undergoes immediately after its translation.

The expression "post-translational modification" refers to the chemical modification of a protein as a result of its translation. Some amino acids of the protein can be modified through the addition of functional groups that are able to expand the overall functionality of the protein. The carbamylation is a non-enzymatic process using the cyanate and is responsible for the modification of the N-terminal part of the proteins due to the conversion of the amino acid lysine to homocitrulline.

Some studies demonstrate that the carbamylation increases under inflammatory and oxidative-stress conditions. The neutrophil-derived myeloperoxidase (MPO) action can lead to high local levels of cyanate, which catalyzes the protein carbamylation. The potential pathological consequences of this process may involve the structural changes of the proteins that may expose novel antigenic epitopes, the disruption of the immunological tolerance and triggering an auto-antibody response specific for the proteins exhibiting the novel post-translational carbamylation modification.

In the present invention, the expressions "carbamylated β2-glycoprotein I" and "carbamylated form of the β2-glycoprotein I" all mean the β2-glycoprotein I protein comprising the post-translational carbamylation modification.

In the present invention, the β2-glycoprotein I may be referred to by the "β2-GPI" acronym. Therefore, the carbamylated β2-glycoprotein I may be referred to as "carbamylated β2-GPI".

The oxidative stress is the main event responsible for structural modifications of the β2-glcoprotein I and its antigenicity in the context of the autoimmune diseases. The diagnostic processes based on the detection of antibodies directed against the oxidized form of β2-glycoprotein I or against the glycated form of said protein do not allow to diagnose the APS in all of the affected subjects. In fact, it has been observed that some subjects having the clinical manifestations of the APS do not have auto-antibodies directed against the oxidized form of the β2-glycoprotein I, nor antibodies directed against the glycated form of said protein. Therefore, when subjected to said diagnostic processes, these individuals test negative. For these cases, the term seronegative APS (SN-APS) has been proposed and is now in use.

According to the present invention, the subjects having the clinical manifestations of the APS but testing negative on all of the currently available diagnostic examinations are referred to as "SN-APS subjects".

Advantageously, it has been observed that the process of the invention allows a diagnosis of APS to be formulated even in SN-APS subjects. In fact, the process of the invention is based on the detection of auto-antibodies for a novel biomarker of APS: the carbamylated β2-glycoprotein I, i.e. provided with the post-translational carbamylation modification. According to the present invention, the β2-glycoprotein I protein in its carbamylated form (i.e., the β2-glycoprotein I protein that has undergone carbamylation) has been identified as specific biomarker of the APS. Therefore, said protein in its carbamylated form can be advantageously used for the diagnosis of APS. According to the process of the invention, the presence of auto-antibodies directed against the carbamylated form of the β2-glycoprotein I in one or more isolated biological samples of the subject to be diagnosed, is capable of defining a positive diagnosis of APS. In other words, when antibodies directed against the carbamylated form of the β2-glycoprotein I are detected within said one or more isolated biological samples of the subject to be diagnosed, said subject is affected by APS. According to the invention, the isolated biological samples of the subject, on which the process of the invention is performed, are derived from blood and thus consist indifferently of serum or plasma. Other biological fluids (urine, synovial fluid, cerebrospinal fluid, and saliva) may also be analyzed. In a preferred embodiment, the isolated biological samples of the subject according to step a) of the process of the invention are serum samples.

Step b) of the process according to the invention is preferably carried out by ELISA assay (*enzyme-linked immunosorbent assay*) or by chemiluminescence assay CLIA (*Chemiluminescent immunoassay*). The ELISA assay is a method of immunoassay used in biochemistry to detect the presence in a sample of a substance through the use of one or more antibodies to one of which an enzyme is bound.

The ELISA assay is also widely used to ascertain the presence of antibodies against a specific antigen in the serum or blood plasma of the subject from which the sample is isolated. The ELISA assay, in which the presence of antibodies in a serum or plasma sample isolated against a specific antigen is detected, is known as indirect ELISA. In the indirect ELISA assay, the presence (in a sample isolated from a subject) of antibodies against an antigen is assessed. According to the present invention, said antigen is the carbamylated β2-glycoprotein I protein. Therefore, in order to assess the presence of antibodies directed against the carbamylated β2-glycoprotein I in one or more isolated biological samples of the subject, the carbamylated β2-glycoprotein I is absorbed on a solid phase. To perform the ELISA assay, the carbamylated β2-glycoprotein I protein absorbed on a solid phase is achieved according to known techniques (see Example 1).

The isolated serum sample of the subject under diagnosis is incubated with said solid phase (on which the carbamylated form of the β2-glycoprotein I is absorbed) and any immune complex formed (comprised of the carbamylated form of the β2-glycoprotein I and of the carbamylated β2-glycoprotein I antibody possibly present in the isolated serum of the subject) is detected by a secondary antibody tagged with an enzyme selected from alkaline phosphatase and peroxidase, said secondary antibody binding to the carbamylated anti-β2-glycoprotein I antibody possibly present in the serum of the subject diagnosed. At this point, we add the substrate of said enzyme selected from alkaline phosphatase and peroxidase and, in case of binding of the secondary antibody to the carbamylated anti-β2-glycoprotein I antibody, there will be staining by reaction between the enzyme bound to said secondary antibody and its substrate. In other words, the staining is present only if the secondary antibody tagged with an enzyme selected from alkaline phosphatase and peroxidase will be bound to the carbamylated anti-β2-glycoprotein I antibody possibly present in the isolated serum of the subject diagnosed. The activity measured is directly proportional to the amount of carbamylated anti-β2-glycoprotein I antibody present in the isolated serum sample of the subject to be diagnosed.

In fact, advantageously, the process of the invention also allows for the assessment of the titre of the antibodies specific for the carbamylated β2-glycoprotein I.

The antibody titre is a measure of the antibodies produced by the body against a specific antigen. According to the invention, the process to perform an *in vitro* diagnosis of antiphospholipid antibody syndrome (APS) may comprise an additional step c) of quantifying the antibodies directed against the carbamylated β2-glycoprotein I that are detected according to step b). Said step c) is performed downstream of step b) and may be optional. In fact, the mere detection of antibodies directed against the carbamylated β2-glycoprotein I in one or more isolated samples from the subject (step b) is able of providing a diagnosis of APS.

Advantageously, step c) of quantifying the concentration of the antibodies against the carbamylated β2-glycoprotein I, which may be associated with arterial and venous thrombosis and/or abortive first-trimester pregnancy, can be performed in order to estimate the severity of antiphospholipid antibody syndrome (APS), even in SN-APS subjects, and to select the most appropriate pharmacological treatment.

The quantification step c) of the antibodies against the carbamylated β2-glycoprotein I can also be performed by chemiluminescence assay. In the chemiluminescence assay the antigen of interest (in the present case the carbamylated form of β2-glycoprotein I) is immobilized on the surface of magnetic beads.

The detection of the immunological reaction between the antigen immobilized on the surface of said magnetic beads and the antibodies present in the isolated serum sample of the subject, is based on a chemical reaction that causes the emission of light. In particular, the presence of possible carbamylated anti-β2-glycoprotein I antibodies present in the isolated sample of the subject and which have bound to carbamylated β2-glycoprotein I can be detected through the use of secondary antibodies tagged with an enzyme that allows obtaining a luminescence in the final step. The addition of the substrate of said enzyme produces light. When step b) of the process of the invention is carried out by chemiluminescence assay, step c) of quantifying any antibodies possibly present in the isolated sample from the subject may be performed by detecting the amount of emitted light. In fact, the intensity of the emitted light is indicative of the amount of carbamylated anti-β2-glycoprotein I antibodies possibly present in the isolated biological sample of the subject.

In another embodiment, step b) of the process according to the invention is carried out by chemiluminescence or however by particle-based multi-analyte technologies based on paramagnetic antigen-coupled particles (PMAT). The diluted serum is blended with the paramagnetic particles, which are pre-coupled with the specific antigen. The beads are transferred in a chamber, where two diodes will illuminate the beads. One diode will identify the particles that have bound to the antibodies and the second diode will determine the *magnitude* of the phycoerythrin signal. A high-resolution device will capture and represent the image.

In another embodiment, step b) of the process according to the invention and the further step c) may be carried out by dot blot or assays using "protein arrays".

The process of the invention is a non-invasive process, which allows to perform, in a short time, a diagnosis of antiphospholipid antibody syndrome (APS) and to select the most suitable pharmacological treatment for the patient, which treatment very often must be timely, in order to prevent the occurrence of widespread thrombotic episodes with multi-organ involvement, as well as diseases of pregnancy and spontaneous abortions.

A further object of the present invention is a kit for the *in vitro* diagnosis of the antiphospholipid antibody syndrome, comprising at least one carbamylated β2-glycoprotein I adsorbed on solid phase, at least one secondary antibody tagged with an enzyme and at least one substrate of said enzyme.

The secondary antibody present in the kit of the invention is an antibody capable of recognizing and binding the carbamylated anti-β2-glycoprotein I antibody, possibly present in the isolated serum sample of the subject to be diagnosed.

In a particularly preferred embodiment, the kit of the invention is a kit for an ELISA assay, preferably for an indirect ELISA assay. In said embodiment, the kit of the invention comprises at least one carbamylated β2-glycoprotein I protein absorbed on a solid phase, at least one secondary antibody tagged with an enzyme. The enzyme conjugated to the secondary antibody can be the alkaline phosphatase which has p-nitrophenyl phosphate (pNPP) as a substrate, or the peroxidase which uses o-phenylenediamine (OPD) and possibly the compound 3,3',5,5'-tetramethylbenzidine (TMB) as substrates.

In an embodiment, the solid phase on which the carbamylated β2-glycoprotein I protein is absorbed is the well of a plate commonly used in the laboratory. Therefore, in an embodiment, the kit according to the invention may comprise a plate on which a carbamylated β2-glycoprotein I is absorbed at one or more wells of said plate, at least one secondary antibody tagged with an enzyme and at least one substrate of said enzyme.

The kit of the invention may comprise additional means to perform an ELISA-type assay.

In an alternative embodiment, the kit of the invention is a kit for a chemiluminescence assay. Said kit comprises at least one carbamylated β2-glycoprotein I protein absorbed on solid phase (magnetic beads) and at least one dimethyl acridinium ester (DMAE) conjugate. In this embodiment, the addition of two priming solutions A (hydrogen peroxide) and B (an alkaline solution) generates an oxidation of the ester to an electronically excited form that allows to achieve a luminescence in the final step. The light emission signal, measured and expressed in Relative Light Units (RLU), is directly proportional to the concentration of specific auto-antibodies present in the tested sample.

Advantageously, the kit of the invention, preferably a kit for an ELISA assay, allows to perform a non-invasive diagnosis of antiphospholipid antibody syndrome (APS) for a subject affected by said syndrome thanks to the detection of antibodies directed against a specific biomarker of said syndrome.

### DESCRIPTION OF THE FIGURES

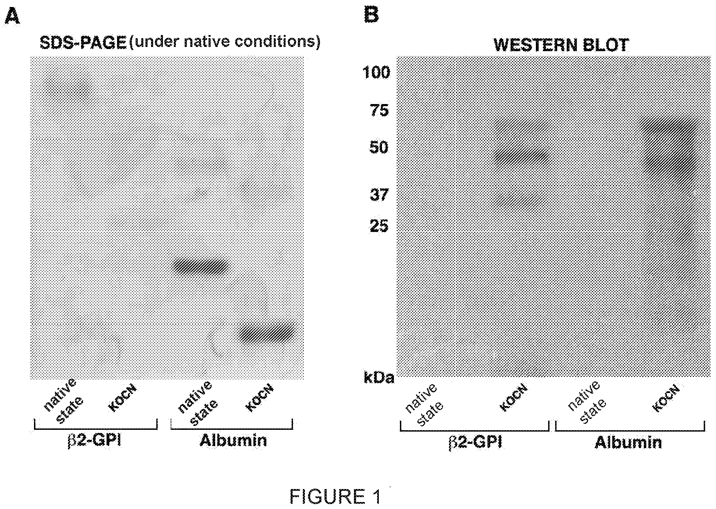

Figure 1. Post-translational modifications of the β2-GPI induced by potassium cyanate (KOCN). (A) Analysis by SDS-PAGE (under native conditions) and staining with Coomassie SimplyBlue of β2-GPI after carbamylation with KOCN.

(B) Western blot analysis of β2-GPI with carbamylated anti-lysine antibodies after carbamylation with KOCN.

As a control, a sample of human albumin treated with KOCN is shown.

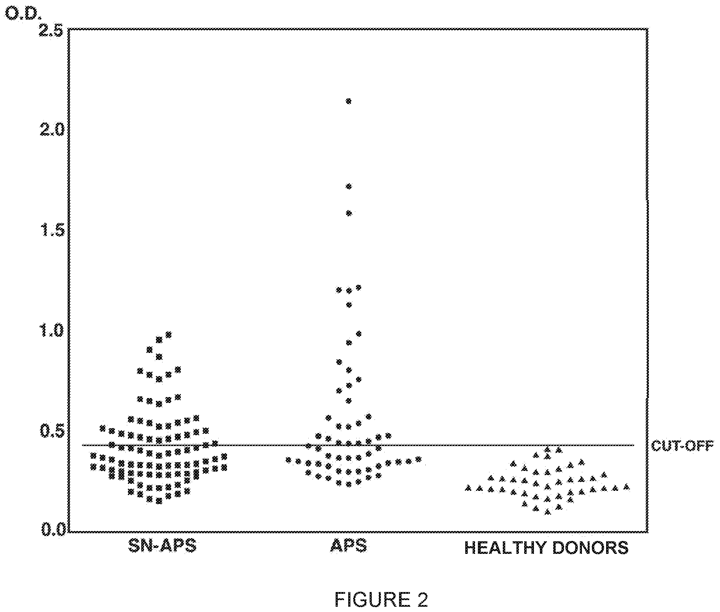

Figure 2. Dosage of carbamylated anti-β2-GPI antibody in patients and healthy subjects. The sera from patients with SN-APS (114), APS (60) and healthy subjects (50) were analyzed by ELISA for the detection of carbamylated anti-β2-GPI antibodies. The presence of carbamylated anti-β2-GPI IgG were significantly higher in the patients with APS and SN-APS than in healthy donors (p<0.001).

### EXPERIMENTAL SECTION

### Example 1

### Incubation of serum samples isolated from subjects with carbamylated β2-GPI

In order to evaluate the use of the carbamylated β2-GPI protein as a biomarker of the anti phospholipid antibody syndrome, an experimental study was performed that comprised the following steps:
a) preparation of the carbamylated β2-GPI and incubation on a solid support;
b) incubation of serum samples with carbamylated β2-GPI;
c) determination of the titre of the antibodies possibly present in the serum samples.

In particular, the β2-GPI supplied by RayBiotech, Peachtree Comers, GA, USA, was carbamylated by using potassium cyanate (KOCN; Merck, Milan, Italy). In particular, 1 mg/ml of the protein was mixed with KOCN at 1M concentration and incubated at 37°C for 12 hours. Next, the sample is dialyzed against H₂O. As a control, a highly purified preparation of human albumin treated with KOCN under the same conditions was used. To verify the presence of carbamylated lysines in the KOCN-treated β2-GPI protein, the samples were analyzed by SDS-PAGE on a 10% gel under native conditions and after staining with Coomassie Simply Blue (Sigma-Aldrich, Milan, Italy). It was also possible to analyze the presence of carbamylated lysines in KOCN-treated β2-GPI protein by means of Western blot analysis by using carbamylated anti-Lysine polyclonal antibodies (e.g., provided by Clini Sciences, Nanterre, France). The resulting carbamylated β2-GPI was added to a solid medium (preferably a 96-well ELISA plate). In particular, each well of the plate was incubated for 8-12 hours at 4°C with 100 µl of a 10 µg/ml solution of carbamylated β2-GPI in PBS. After 3 washes with phosphate buffered saline (PBS) containing 0.05% Tween-20 (PBS-T), the wells were incubated for 2 hours at room temperature with 100 µl/well of blocking buffer (PBS containing 3% bovine serum albumin, BSA; Sigma-Aldrich) and subj ected to 3 washes with PB S-T.

The wells of the ELISA plate were incubated with the patient sera, diluted 1:100 (5 µl in 495 µl of blocking buffer). Rabbit polyclonal antibodies specific for the carbamylated lysine residues (CliniSciences, Nanterre, France) are used as a positive control.

The titre of the antibodies present in the samples subj ected to ELISA assay was analyzed by incubating the plate, after 3 additional washes with PBS-T, for 1 hour at room temperature, with secondary human anti-IgG antibodies (for the human sera) or, alternatively, with peroxidase-conjugated rabbit anti-IgG (as positive control) (Sigma-Aldrich), diluted 1:1000 in the blocking buffer (PBS containing 3% bovine serum albumin).

The plates were washed 3 times as set forth above. Peroxidase binding was detected by adding 100 µl/well of a solution of O-phenylenediamine chloride, a substrate of said peroxidase, and incubating the samples with said solution for 10 minutes at room temperature. The reaction was then blocked with 50 µl/well of IN H₂SO₄, for 5 min. The absorbance was read at 492 nm in an ELISA plate reader.

The data are presented as an average of the optical density (OD) achieved. Fifty sera from healthy donors were tested to determine a cut-off value (value above which a result is considered positive) that was established as the mean OD ± 3 SD (Standard Deviation) of the sera from healthy donors.

## Claims

1. A process to perform an *in vitro* diagnosis of the antiphospholipid antibody syndrome (APS) comprising the steps of:
a) having available one or more isolated biological samples that have been obtained from the subject to be diagnosed; and
b) identifying, within said one or more samples according to step a), the presence of antibodies directed towards the carbamylated β2-glycoprotein I.

2. The process according to claim 1, wherein said carbamylated p2-glycoprotein I is made by the post-translation modification by carbamylation of the β2-glycoprotein I.

3. The process according to claim 1, also comprising the further step c) of quantifying the antibodies directed towards the carbamylated β2-glycoprotein I, which are identified according to step b).

4. The process according to claim 1, wherein said step b) is carried out by means of ELISA assay or chemiluminescence assay.

5. The process according to claim 1, wherein said step b) is carried out by means of particle-based multi-analytical technologies.

6. The process according to claim 1, wherein said one or more isolated biological samples of the subject to be diagnosed according to step a) are samples selected from serum, plasma, urine, synovial fluid, cerebrospinal fluid, saliva.

7. The process according to the preceding claim, wherein said one or more isolated biological samples of said subject, are isolated samples of serum.

8. A kit for the *in vitro* diagnosis of the antiphospholipid antibody syndrome (APS) comprising at least one carbamylated β2-glycoprotein I adsorbed on solid phase, at least one secondary antibody tagged with an enzyme and at least one substrate of said enzyme.

9. The kit according to claim 8, wherein said enzyme of said secondary antibody is selected from alkaline phosphatase and peroxidase, and said at least one substrate of said enzyme is selected from p-nitrophenyl phosphate (pNPP) or o-phenylenediamine (OPD), respectively.

10. Use of antibodies directed against the carbamylated β2-glycoprotein I as biomarkers of the antiphospholipid antibody syndrome (APS).

## Patentansprüche

1. Ein Verfahren zur Durchführung einer *In*-*vitro*-Diagnose des Antiphospholipid-Antikörper-Syndroms (APS), das die Schritte umfasst:
a) Bereitstellen einer oder mehrerer isolierter biologischer Proben, die aus einem zu diagnostizierenden Subjekt erhalten worden sind; und
b) Identifizieren des Vorhandenseins von Antikörpern gegen das carbamylierte β2-Glykoprotein I in dieser einen oder mehreren Proben gemäß Schritt a).

2. Verfahren nach Anspruch 1, wobei das carbamylierte β2-Glykoprotein I durch posttranslationale Modifikation mittels Carbamylierung des β2-Glykoproteins I hergestellt wird.

3. Das Verfahren gemäß Anspruch 1, das außerdem den weiteren Schritt c) der Quantifizierung der gegen das carbamylierte β2-Glykoprotein I gerichteten Antikörper umfasst, die gemäß Schritt b) identifiziert wurden.

4. Das Verfahren gemäß Anspruch 1, wobei der Schritt b) mit Hilfe von ELISA-Test oder Chemilumineszenztest durchgeführt wird.

5. Das Verfahren nach Anspruch 1, wobei der Schritt b) mit Hilfe von teilchenbasierter Multianalysetechnologien durchgeführt wird.

6. Das Verfahren gemäß Anspruch 1, wobei die eine oder mehreren isolierten biologischen Proben des gemäß Schritt a) zu diagnostizierenden Subjekts Proben sind, die aus Serum, Plasma, Urin, Synovialflüssigkeit, Zerebrospinalflüssigkeit oder Speichel ausgewählt sind.

7. Das Verfahren gemäß dem vorhergehenden Anspruch, wobei die eine oder mehreren isolierten biologischen Proben des Subjekts isolierte Serumproben sind.

8. Ein Kit für die *In*-*vitro*-Diagnose des Antiphospholipid-Antikörper-Syndroms (APS), umfassend mindestens ein an einer Festphase adsorbiertes carbamyliertes β2-Glykoprotein I, mindestens einen mit einem Enzym markierten Sekundärantikörper und mindestens ein Substrat des genannten Enzyms.

9. Das Kit gemäß Anspruch 8, wobei das Enzym des sekundären Antikörpers aus alkalischer Phosphatase und Peroxidase ausgewählt ist und das mindestens eine Substrat des Enzyms aus p-Nitrophenylphosphat (pNPP) beziehungsweise o-Phenylendiamin (OPD) ausgewählt ist.

10. Verwendung von Antikörpern gegen das carbamylierte β2-Glykoprotein I als Biomarker des Antiphospholipid-Antikörper-Syndroms (APS).

## Revendications

1. Procédé pour effectuer un diagnostic *in vitro* du syndrome des antiphospholipides (APS) comprenant les étapes consistant à :
a) disposer d'un ou plusieurs échantillons biologiques isolés provenant du sujet à diagnostiquer ; et
b) identifier, dans ledit ou lesdits échantillons selon l'étape a), la présence d'anticorps dirigés contre la β2-glycoprotéine I carbamylée.

2. Procédé selon la revendication 1, dans lequel ladite β2-glycoprotéine I carbamylée est produite par la modification post-traductionnelle par carbamylation de la β2-glycoprotéine I.

3. Procédé selon la revendication 1, comprenant également l'étape supplémentaire c) de quantification des anticorps dirigés contre la β2-glycoprotéine I carbamylée, qui sont identifiés selon l'étape b).

4. Procédé selon la revendication 1, dans lequel ladite étape b) est réalisée au moyen d'un test ELISA ou d'un test de chimiluminescence.

5. Procédé selon la revendication 1, dans lequel ladite étape b) est réalisée au moyen de technologies multi-analytiques basées sur les particules.

6. Procédé selon la revendication 1, dans lequel ledit ou lesdits échantillons biologiques isolés du sujet à diagnostiquer selon l'étape a) sont des échantillons choisis parmi le sérum, le plasma, l'urine, le liquide synovial, le liquide céphalo-rachidien, la salive.

7. Procédé selon la revendication précédente, dans lequel lesdits échantillons biologiques isolés du sujet sont des échantillons isolés de sérum.

8. Trousse pour le diagnostic in vitro du syndrome des antiphospholipides (APS) comprenant au moins une β2-glycoprotéine I carbamylée adsorbée sur phase solide, au moins un anticorps secondaire marqué par une enzyme et au moins un substrat de ladite enzyme.

9. Trousse selon la revendication 8, dans lequel l'enzyme de l'anticorps secondaire est choisie parmi la phosphatase alcaline et la peroxydase, et ledit au moins un substrat de ladite enzyme est choisi parmi le p-nitrophénylphosphate (pNPP) ou l'o-phénylènediamine (OPD), respectivement.

10. Utilisation d'anticorps dirigés contre la β2-glycoprotéine I carbamylée comme biomarqueurs du syndrome des antiphospholipides (APS).
